# EUROPÄISCHE PATENTSCHRIFT

(11) **EP 4 129 203 B1**
(45) Veröffentlichungstag und Bekanntmachung des Hinweises auf die Patenterteilung: **11.11.2026**
(21) Anmeldenummer: 21190196.2
(22) Anmeldetag: 06.08.2021
(51) Int. Cl.: A61B 17/02

(54) **CHIRURGISCHER RETRAKTOR FÜR DIE HERZCHIRURGIE**
SURGICAL RETRACTOR FOR CARDIAC SURGERY
RÉTRACTEUR CHIRURGICAL POUR LA CHIRURGIE CARDIAQUE

(43) Veröffentlichungstag der Anmeldung: 08.02.2023
(73) Patentinhaber: Fehling Instruments GmbH, 63791 Karlstein (DE)
(72) Erfinder: Fehling, Gerald, 63755 Alzenau (DE); Treede, Hendrik, 53177 Bonn (DE)
(74) Vertreter: Westphal, Mussgnug & Partner Patentanwälte mbB

(56) Entgegenhaltungen:
- WO-A1-01/06934
- US-A1- 2011 275 902
- US-A1- 2018 317 900

## Beschreibung

Die Erfindung betrifft einen chirurgischen Retraktor für die Herzchirurgie.

Die EP 2 124 757 B1 offenbart einen Vorhofretraktor zur Verwendendung in einem chirurgischen Eingriff an einem Herzen, bei welchem ein Retraktorblatt an einem distalen Ende eines Handgriffs angeordnet ist, und wobei an dem Retraktorblatt eine schwenkbare Klappe angeordnet ist, die zur Vergrößerung der Anlagefläche ausgeschwenkt werden kann. Um die Klappe auszuschwenken, wird entsprechender Platz benötigt.

Als weiterer Stand der Technik wird die WO 01/06934 A1 genannt.

Die Aufgabe der Erfindung besteht daher darin, einen chirurgischen Retraktor für die Herzchirurgie bereitzustellen, welcher nach Einführen durch einen Zugang auf einfache Art und Weise im Hinblick auf seine Rückhaltefläche vergrößert werden kann.

Die Aufgabe wird erfindungsgemäß gelöst durch einen chirurgischen Retraktor für die Herzchirurgie mit den Merkmalen des Patentanspruchs 1.

Vorteilhafte Ausgestaltungen und Weiterbildungen der Erfindung sind in den abhängigen Ansprüchen angegeben.

Der erfindungsgemäße chirurgischer Retraktor für die Herzchirurgie weist einen hohlrohrförmigen ersten Grundkörper mit einer ersten Grundkörperlängsachse, an welchem wenigstens eine erste Lamelle aufweisend ein erstes Ende, ein zweites Ende und eine erste Lamellenlängsachse mit ihrem ersten Ende seitlich angeordnet ist, und einen zweiten Grundkörper mit einer zweiten Grundkörperlängsachse, an welchem wenigstens eine zweite Lamelle aufweisend ein erstes Ende, ein zweites Ende und eine zweite Lamellenlängsachse mit ihrem ersten Ende seitlich angeordnet ist, auf, wobei in dem ersten Grundkörper eine erste Transportstange mit einer ersten Transportstangenlängsachse um die erste Transportstangenlängsachse drehbar gelagert angeordnet ist, wobei die wenigstens eine zweite Lamelle den ersten Grundkörper in einer Durchgangsöffnung quer zu der ersten Grundkörperlängsachse durchgreift und wobei die wenigstens eine zweite Lamelle mehrere entlang der zweiten Lamellenlängsachse beabstandet zueinander angeordnete Ausnehmungen und die erste Transportstange wenigstens einen mit den Ausnehmungen zusammenwirkenden Vorsprung aufweist.

Eine derartige Ausgestaltung ermöglicht eine seitliche Verbreiterung der Anlagefläche des Retraktors, welche durch die ersten und zweiten Lamellen aufgespannt wird, insbesondere ohne dass Raum für eine raumgreifende Schwenkbewegung vorgesehen werden muss. Bei Drehen der Transportstange um ihre Transportstangenlängsachse wird aufgrund des Zusammenwirkens des an der Transportstange angeordneten Vorsprungs mit den Ausnehmungen der wenigstens einen zweiten Lamelle die zweite Lamelle durch die Durchgangsöffnung bewegt, insbesondere quer zur ersten Grundkörperlängsachse. Liegt beispielsweise in einer ersten Position der zweite Grundkörper an dem ersten Grundkörper an, kann durch Drehen der Transportstange die wenigstens eine zweite Lamelle seitlich ausgefahren werden, wobei sich der Abstand zwischen dem zweiten Grundkörper und dem ersten Grundkörper vergrößert. Dabei wird auch die Anlagefläche des Retraktors vergrößert.

Sofern im Folgenden von "Lamellen" die Rede ist, sollen soweit nicht explizit anders erwähnt sowohl die ersten als auch die zweiten und soweit vorhanden auch die dritten oder weitere Lamellen umfasst sein. Gleiches gilt auch soweit nicht explizit anders erwähnt für die Verwendung der Bezeichnungen "Grundkörper" und "Transportstangen".

Um eine verbesserte Reinigung zu ermöglichen, können die Ausnehmungen in der wenigstens einen zweiten Lamelle als durchgehende Ausnehmungen ausgebildet sein. Ferner können auch an der wenigstens einen ersten Lamelle entsprechende Ausnehmungen angeordnet sein.

Vorzugsweise weist die erste Transportstange mehrere Vorsprünge auf, welche insbesondere nach Art eines Zahnrads ausgebildet sind. Eine derartige Ausgestaltung kann auf konstruktiv einfache Art und Weise einen zuverlässigen und einfach zu reinigenden Transportmechanismus ermöglichen.

Eine vorteilhafte Weiterbildung der Erfindung sieht vor, dass die erste Transportstange einen unrunden Betätigungsabschnitt, vorzugsweise einen Betätigungsabschnitt mit einem sechseckigen Querschnitt, aufweist, wobei vorzugsweise der Betätigungsabschnitt an einer Stirnseite des ersten Grundkörpers aus dem Grundkörper hervorragt. Ein derartiger Betätigungsabschnitt kann auf einfache Art und Weise eine Drehung der Transportstange durch ein entsprechendes Werkzeug, beispielsweise nach Art eines Schraubenschlüssels, erlauben.

Gemäß einer bevorzugten Ausgestaltung der Erfindung weist der erste Grundkörper drei entlang der ersten Grundkörperlängsachse beabstandet angeordnete erste Lamellen auf. Dabei können die ersten Lamellen an ihrem zweiten Ende mittels eines Querstegs miteinander verbunden sein. Eine derartige rahmenartige Ausgestaltung ermöglicht eine möglichst große Stabilität des Retraktors.

Vorzugsweise weist der zweite Grundkörper zwei entlang der zweiten Grundkörperlängsachse beabstandet angeordnete zweite Lamellen auf. Diese greifen vorzugsweise jeweils in den Zwischenraum zwischen jeweils zwei ersten Lamellen ein. Eine derartige Ausgestaltung kann eine hohe Stabilität bei einem möglichst kompakten Aufbau ermöglichen.

Vorteilhafterweise ist während einer Transportbewegung eine Seitenkante der wenigstens einen zweiten Lamelle entlang einer Seitenkante der wenigstens einen ersten Lamelle gleitend geführt, vorzugsweise nach Art einer Spundung. Dadurch kann die Stabilität auch während einer Transportbewegung erhöht werden.

Eine bevorzugte Weiterbildung der Erfindung sieht vor, dass der zweite Grundkörper eine Länge aufweist, welche kleiner als eine Länge des ersten Grundkörpers ist, vorzugsweise um wenigstens 10% der Länge des ersten Grundkörpers. Dadurch kann der Retraktor besser an die anatomischen Gegebenheiten im Herzen angepasst werden.

Gemäß einer besonders bevorzugten Weiterbildung der Erfindung ist ein dritter Grundkörper mit einer dritten Grundkörperlängsachse vorgesehen, an welchem wenigstens eine dritte Lamelle aufweisend ein erstes Ende, ein zweites Ende und eine dritte Lamellenlängsachse mit ihrem ersten Ende seitlich angeordnet ist, wobei der zweite Grundkörper hohlrohrförmig ausgebildet ist und in dem zweiten Grundkörper eine zweite Transportstange mit einer zweiten Transportstangenlängsachse um die zweite Transportstangenlängsachse drehbar gelagert angeordnet ist, wobei die wenigstens eine dritte Lamelle den zweiten Grundkörper in einer Durchgangsöffnung quer zu der zweiten Grundkörperlängsachse durchgreift und wobei die wenigstens eine dritte Lamelle mehrere entlang der dritten Lamellenlängsachse beabstandet zueinander angeordnete Ausnehmungen und die zweite Transportstange wenigstens einen mit den Ausnehmungen zusammenwirkenden Vorsprung aufweist. Ein derartiger dritter Grundkörper ermöglicht eine weitere Verbreiterung der Anlagefläche des Retraktors. Dabei lässt sich der Retraktor zum Einführen durch einen Zugang jedoch derart zusammenschieben, dass seine Breite im Wesentlichen der Länge der ersten Lamelle zuzüglich der Breite der Grundkörper entspricht. Die Breite im vollständig auseinandergeschobenen Zustand kann damit im Vergleich zu der Breite im vollständig zusammengeschobenen Zustand in etwa verdreifacht werden.

In analoger Weise können auch ein vierter Grundkörper und gegebenenfalls weitere Grundkörper vorgesehen werden.

Vorzugsweise weist der dritte Grundkörper zwei entlang der dritten Grundkörperlängsachse beabstandet angeordnete dritte Lamellen auf, um eine möglichst ausreichende Anlagefläche aufspannen zu können.

Gemäß einer vorteilhaften Ausgestaltung der Erfindung entspricht die Breite einer Lamelle etwa der Länge des ersten Grundkörpers geteilt durch die Anzahl sämtlicher Lamelle des Retraktors. Mit anderen Worten wird vorzugsweise die Breite einer Lamelle so groß wie möglich gewählt, um eine möglichst hohe Stabilität und eine möglichst große Anlagefläche ermöglichen zu können.

Eine besonders bevorzugte Ausgestaltung der Erfindung sieht vor, dass die Lamellen entlang ihrer Lamellenlängsachse gekrümmt ausgebildet sind. Eine derartige Ausgestaltung ermöglicht eine gute Anpassung des Retraktors an die anatomischen Gegebenheiten.

Gemäß einer vorteilhaften Ausführungsform der Erfindung weist die Durchgangsöffnung eine gekrümmte Anlagefläche für die Lamelle auf, wodurch die Stabilität des Retraktors erhöht und die Führung der Lamellen während des Transport verbessert werden kann..

Vorzugsweise weisen die Lamellen an ihrem zweiten Ende ein vorzugsweise elastisch ausgebildetes Anschlagelement auf. Das Anschlagelement verhindert ein ungewolltes Herausdrehen der Lamellen aus der Durchgangsöffnung des jeweiligen Grundkörpers. Ist das Anschlagelement elastisch ausgebildet, kann dies ein gewolltes Herausdrehen, indem bewusst eine höhere Kraft aufgewendet wird, beispielsweise zum Zerlegen des Retraktors zu Reinigungszwecken oder bei Austausch von Lamellen, ermöglichen.

Vorteilhafterweise weisen die hohlrohrförmigen Grundkörper wenigstens eine Spülöffnung, vorzugsweise mehrere Spülöffnungen, auf, um eine verbesserte Reinigbarkeit zu ermöglichen.

Besonders bevorzugt ist an dem ersten Grundkörper ein Aufnahmeelement zur Aufnahme eines Halte- und/oder Manipulationsgriffs angeordnet. Mittels des Halte- und/oder Manipulationsgriffs kann der Retraktor während eines chirurgischen Eingriffs durch einen Zugang eingesetzt und in einer gewünschten Position und/oder Ausrichtung positioniert werden.

Der erfindungsgemäße chirurgische Retraktor kommt insbesondere in der Herzchirurgie zur Anwendung.

Ein Ausführungsbeispiel der Erfindung wird anhand der nachfolgenden Figuren ausführlich erläutert. Es zeigen
- Figur 1: eine perspektivische Ansicht eines Ausführungsbeispiels eines erfindungsgemäßen chirurgischen Retraktors für die Herzchirurgie,
- Figur 2: eine weitere perspektivische Ansicht des Retraktors gemäß Figur 1,
- Figur 3: eine Draufsicht auf den Retraktor gemäß Figur 1,
- Figur 4: einen Schnitt entlang der Linie A-A in Figur 3,
- Figur 5: eine Ausschnittvergrößerung aus Figur 4,
- Figur 6: eine perspektivische Ansicht der beiden Transportstangen des Retraktors gemäß Figur 1 und
- Figur 7: den Retraktor gemäß Figur 1 mit einem daran angeordneten Halte- und/oder Manipulationsgriff.

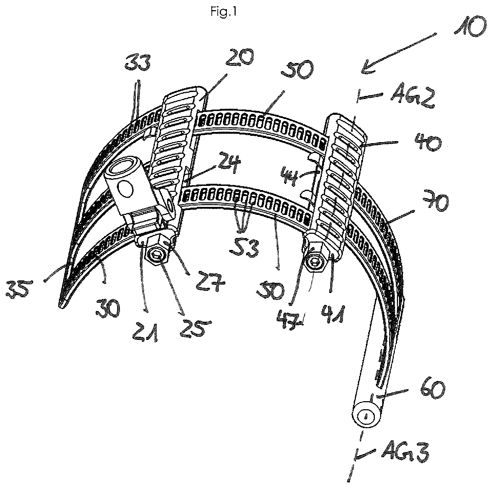

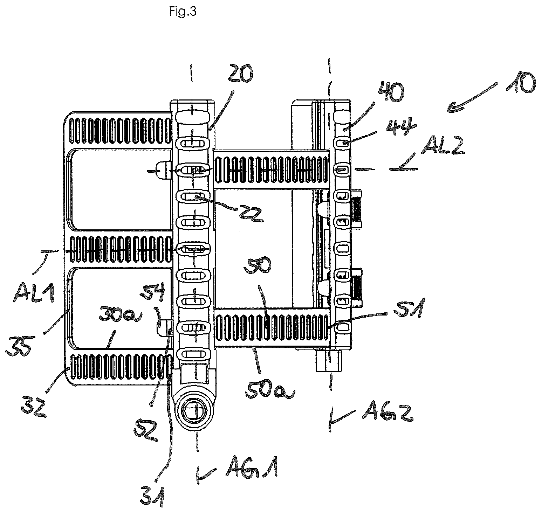

Die Figuren 1 bis 7 zeigen verschiedene Ansichten eines Ausführungsbeispiels eines erfindungsgemäßen chirurgischen Retraktors 100 für die Herzchirurgie. Gleiche Bezugsziffern bezeichnen gleiche oder funktionsgleiche Teile, wobei zur besseren Übersicht nicht sämtliche Bezugsziffern in sämtlichen Figuren angegeben sind.

Der chirurgischer Retraktor 10 für die Herzchirurgie weist einen hohlrohrförmigen ersten Grundkörper 20 mit einer ersten Grundkörperlängsachse AG1 auf. Der ersten Grundkörper 20 kann aus zwei Halbschalen 20a, 20b zusammengesetzt sein, was die Herstellung und Montage vereinfachen kann. An dem ersten Grundkörper 20 ist wenigstens eine erste Lamelle 30, im vorliegenden Ausführungsbeispiel drei erste Lamellen 30, aufweisend ein erstes Ende 31, ein zweites Ende 32 und eine erste Lamellenlängsachse AL1 mit ihrem ersten Ende 31 seitlich angeordnet. Die seitliche Anordnung führt insbesondere dazu, dass die erste Lamellenlängsachse AL1 im Wesentlichen quer, beispielsweise im Wesentlichen senkrecht, zu der ersten Grundkörperlängsachse AG1 angeordnet ist. Die zweiten Enden 32 der ersten Lamellen 30 können mittels eines Querstegs 35 verbunden sein, um die Stabilität des Retraktors 10 zu verbessern.

Der Retraktor 10 weist weiterhin einen zweiten Grundkörper 40 mit einer zweiten Grundkörperlängsachse AG2 auf, an welchem wenigstens eine zweite Lamelle 50, im vorliegenden Ausführungsbeispiel zwei zweite Lamellen 50, jeweils aufweisend ein erstes Ende 51, ein zweites Ende 52 und eine zweite Lamellenlängsachse AL2 mit ihrem ersten Ende 51 seitlich angeordnet ist. Die seitliche Anordnung führt insbesondere dazu, dass die zweite Lamellenlängsachse AL2 im Wesentlichen quer, beispielsweise im Wesentlichen senkrecht, zu der zweiten Grundkörperlängsachse AG2 angeordnet ist.

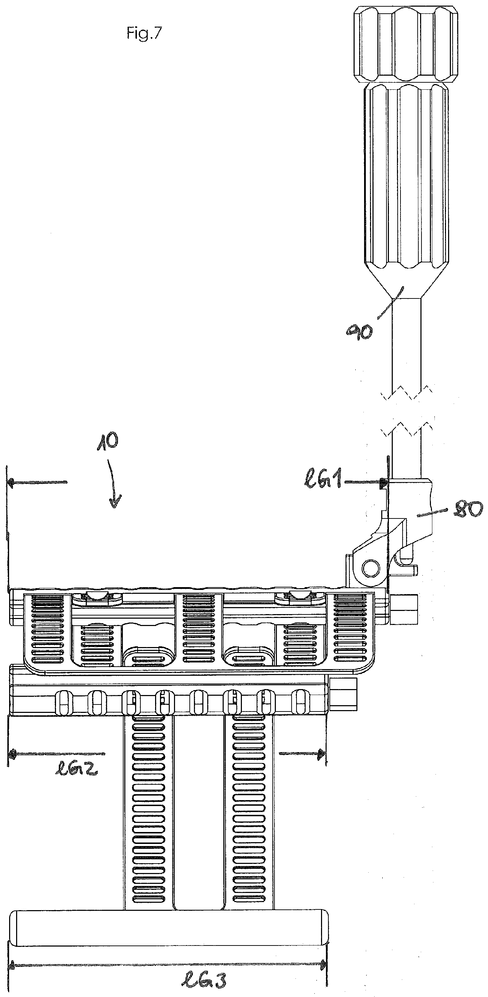

Der zweite Grundkörper 40 kann eine Länge 1G2 aufweisen, welche kleiner als eine Länge 1G1 des ersten Grundkörpers 20 ist, vorzugsweise um wenigstens 10% der Länge 1G1 des ersten Grundkörpers 20 (vgl. Figur 7).

In dem ersten Grundkörper 20 ist eine erste Transportstange 25, welche eine erste Transportstangenlängsachse AT1 aufweist, um die erste Transportstangenlängsachse AT1 drehbar gelagert angeordnet. Der ersten Grundkörper 20 kann aus zwei Halbschalen 20a, 20b zusammengesetzt sein, was die Herstellung und Montage, insbesondere das Einlegen der ersten Transportstange 25 in den ersten Grundkörper 20, vereinfachen kann.

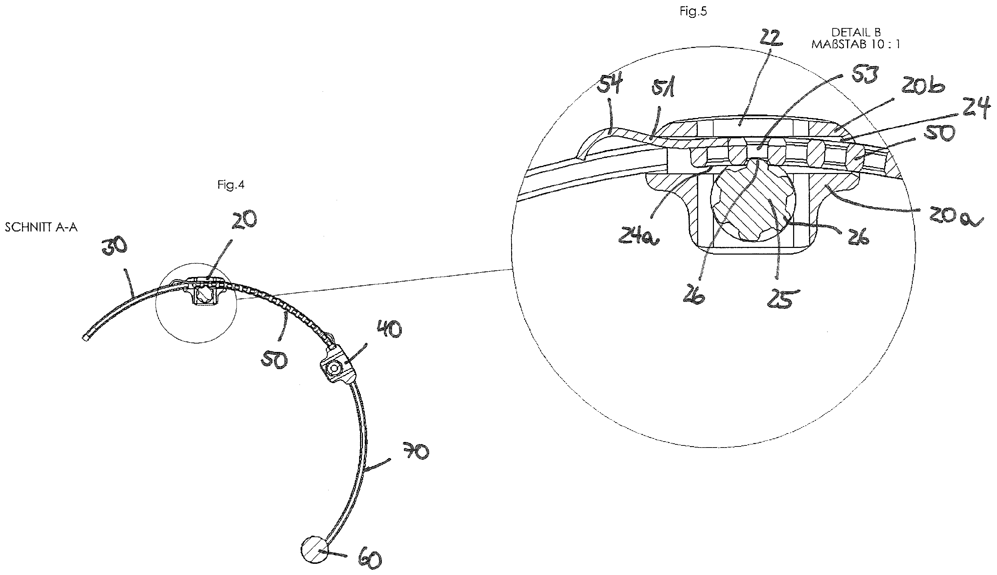

Der erste Grundkörper 20 weist eine Durchgangsöffnung 24 auf, welche insbesondere im Wesentlichen quer zur ersten Grundkörperlängsachse AG1 verläuft. Die wenigstens eine zweite Lamelle 50 ist in der Durchgangsöffnung 24 angeordnet. Insbesondere durchgreift die wenigstens eine zweite Lamelle 50 den ersten Grundkörper 20 in der Durchgangsöffnung 24 quer zu der ersten Grundkörperlängsachse AG1 (vgl. insbesondere Figur 5). Sind wie in dem vorliegenden Ausführungsbeispiel zwei zweite Lamellen 50 vorhanden, weist der erste Grundkörper 20 vorzugsweise zwei Durchgangsöffnungen 24 auf, und in jeder der Durchgangsöffnungen 24 ist eine zweite Lamelle 50 angeordnet.

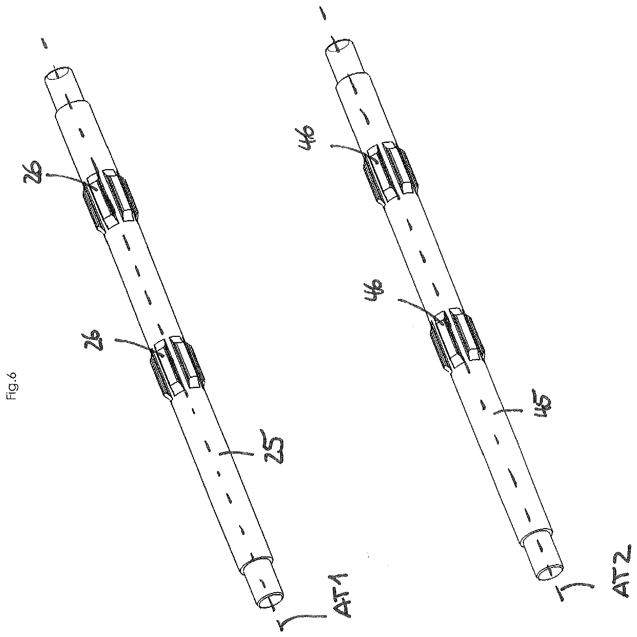

Die wenigstens eine zweite Lamelle 50 weist mehrere entlang der zweiten Lamellenlängsachse AL2 beabstandet zueinander angeordnete Ausnehmungen 53 auf, während die erste Transportstange 25 wenigstens einen mit den Ausnehmungen 53 zusammenwirkenden Vorsprung 26 aufweist. Die Ausnehmungen 53 sind insbesondere nicht lediglich als Vertiefungen, sondern als durchgehende Ausnehmungen 53 ausgebildet. Vorzugsweise weist die erste Transportstange 25 mehrere Vorsprünge 26 auf, welche beispielsweise nach Art eines Zahnrads ausgebildet sind (vgl. Figur 5 und 6). Sind zwei zweite Lamellen 50 vorhanden, weist die erste Transportstange 25 für jede der zweiten Lamellen 50 wenigstens einen Vorsprung 26, vorzugsweise mehrere Vorsprünge 26 nach Art jeweils eines Zahnrads, auf (vgl. Figur 6).

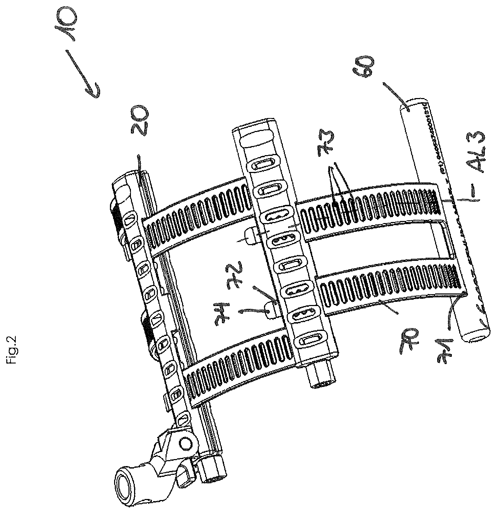

Die erste Transportstange 25 kann einen unrunden Betätigungsabschnitt 27 aufweisen, welcher in dem vorliegenden Ausführungsbeispiel als Betätigungsabschnitt 27 mit einem sechseckigen Querschnitt ausgebildet ist. Der Betätigungsabschnitt 27 kann an einer Stirnseite 21 des ersten Grundkörpers 20 aus dem Grundkörper 20 hervorragen und ist damit einfach zugänglich (vgl. insbesondere Figuren 1 und 2).

Wird die erste Transportstange 25 um ihre erste Transortstangenlängsachse AT1 gedreht, beispielsweise durch Ansetzen und entsprechende Hebelbewegung eines schraubenschlüsselartigen Werkzeugs an dem Betätigungsabschnitt 27, wird je nach Drehrichtung durch Eingriff der Vorsprünge 26 in die Ausnehmungen 53 die zweite Lamelle 50 weiter in die Durchgangsöffnung 24 eingezogen oder aus dieser herausgeschoben. Die zweite Lamelle 50 kann so weit eingezogen werden, bis der zweite Grundkörper 40 an dem ersten Grundkörper 20 anliegt. Um ein ungewolltes Herausdrehen oder Herausschieben der zweiten Lamelle 50 aus der Durchgangsöffnung 24 des ersten Grundkörpers 20 zu verhindern, kann an dem zweiten Ende 52 der zweiten Lamelle 50 ein Anschlagelement 54 angeordnet sein. Ist das Anschlagelement 54 elastisch ausgebildet, beispielsweise wie in dem vorliegenden Ausführungsbeispiel als elastisch federnde Lippe, kann dies ein gewolltes Herausdrehen, indem bewusst eine höhere Kraft aufgewendet wird, ermöglichen.

Während einer Transportbewegung, **d. h.** während des Einziehens oder Herausschiebens der zweiten Lamelle, kann eine Seitenkante 50a der wenigstens einen zweiten Lamelle 50 entlang einer Seitenkante 30a der wenigstens einen ersten Lamelle 30 gleitend geführt sein, beispielsweise nach Art einer Spundung.

Die wenigstens eine erste Lamelle 30 kann ebenfalls mehrere entlang der ersten Lamellenlängsachse AL1 beabstandet zueinander angeordnete Ausnehmungen 33 aufweisen. Die Ausnehmungen 33 sind insbesondere nicht lediglich als Vertiefungen, sondern als durchgehende Ausnehmungen 53 ausgebildet.

Der Retraktor 10 kann weiterhin einen dritten Grundkörper 60 mit einer dritten Grundkörperlängsachse AG3 auf, an welchem wenigstens eine dritte Lamelle 70, im vorliegenden Ausführungsbeispiel zwei dritte Lamellen 70, jeweils aufweisend ein erstes Ende 71, ein zweites Ende 72 und eine dritte Lamellenlängsachse AL3 mit ihrem ersten Ende 71 seitlich angeordnet ist. Die seitliche Anordnung führt insbesondere dazu, dass die dritte Lamellenlängsachse AL3 im Wesentlichen quer, beispielsweise im Wesentlichen senkrecht, zu der dritten Grundkörperlängsachse AG3 angeordnet ist.

Der dritte Grundkörper 60 kann eine Länge lG3 aufweisen, welche in etwa der Länge lG2 des zweiten Grundkörpers 40 entspricht (vgl. Figur 7).

In dem zweiten Grundkörper 40 kann eine zweite Transportstange 45, welche eine zweite Transportstangenlängsachse AT2 aufweist, um die zweite Transportstangenlängsachse AT2 drehbar gelagert angeordnet sein. Auch der zweite Grundkörper 40 kann wie der erste Grundkörper 20 aus zwei Halbschalen zusammengesetzt sein.

Der zweite Grundkörper 40 kann eine Durchgangsöffnung 44 aufweisen, welche insbesondere im Wesentlichen quer zur zweiten Grundkörperlängsachse AG2 verläuft. Die wenigstens eine dritte Lamelle 70 ist in der Durchgangsöffnung 44 angeordnet. Insbesondere durchgreift die wenigstens eine dritte Lamelle 50 den zweiten Grundkörper 40 in der Durchgangsöffnung 44 quer zu der zweiten Grundkörperlängsachse AG2. Sind wie in dem vorliegenden Ausführungsbeispiel zwei dritte Lamellen 70 vorhanden, weist der zweite Grundkörper 40 vorzugsweise zwei Durchgangsöffnungen 44 auf, und in jeder der Durchgangsöffnungen 44 ist eine dritte Lamelle 70 angeordnet.

Die wenigstens eine dritte Lamelle 70 weist mehrere entlang der dritten Lamellenlängsachse AL3 beabstandet zueinander angeordnete Ausnehmungen 73 auf, während die zweite Transportstange 45 wenigstens einen mit den Ausnehmungen 73 zusammenwirkenden Vorsprung 46 aufweist. Insbesondere weist die zweite Transportstange 45 mehrere Vorsprünge 46 auf, welche beispielsweise nach Art eines Zahnrads ausgebildet sind (vgl. Figur 6). Sind zwei dritte Lamellen 70 vorhanden, weist die zweite Transportstange 45 für jede der dritten Lamellen 70 wenigstens einen Vorsprung 46, vorzugsweise mehrere Vorsprünge 46 nach Art jeweils eines Zahnrads, auf (vgl. Figur 6).

Die zweite Transportstange 45 kann einen unrunden Betätigungsabschnitt 47 aufweisen, welcher in dem vorliegenden Ausführungsbeispiel als Betätigungsabschnitt 47 mit einem sechseckigen Querschnitt ausgebildet ist. Der Betätigungsabschnitt 47 kann an einer Stirnseite 41 des zweiten Grundkörpers 40 aus dem Grundkörper 40 hervorragen und ist damit einfach zugänglich (vgl. insbesondere Figuren 1 und 2).

Wird die zweite Transportstange 45 um ihre zweite Transportstangenlängsachse AT2 gedreht, wird wie anhand der ersten Transportstange 25 beschrieben je nach Drehrichtung durch Eingriff der Vorsprünge 46 in die Ausnehmungen 73 die dritte Lamelle 70 weiter in die Durchgangsöffnung 44 eingezogen oder aus dieser herausgeschoben. Die dritte Lamelle 70 kann so weit eingezogen werden, bis der dritte Grundkörper 60 an dem zweiten Grundkörper 40 anliegt. Um ein ungewolltes Herausdrehen oder Herausschieben der zweiten Lamelle 70 aus der Durchgangsöffnung 44 des zweiten Grundkörpers 40 zu verhindern, kann an dem zweiten Ende 72 der dritten Lamelle 70 ein Anschlagelement 74 angeordnet sein (vgl. Figur 2). Ist das Anschlagelement 74 elastisch ausgebildet, beispielsweise wie in dem vorliegenden Ausführungsbeispiel als elastisch federnde Lippe, kann dies ein gewolltes Herausdrehen, indem bewusst eine höhere Kraft aufgewendet wird, ermöglichen.

Die Breite einer Lamelle 30, 50, 70 entspricht vorzugsweise etwa der Länge lG1 des ersten Grundkörpers 20 geteilt durch die Anzahl sämtlicher Lamellen 30, 50, 70 des Retraktors 10. Dadurch kann die Breite einer Lamelle 30, 50, 70 möglichst groß gewählt werden.

Sind sowohl der zweite Grundkörper 40 anliegend an dem ersten Grundkörper 20 und der dritte Grundkörper 60 anliegend an dem zweiten Grundkörper 40 angeordnet, ragen die zweiten Lamellen 50 und die dritten Lamellen 70 in die Zwischenräume zwischen den ersten Lamellen 30. Besonders bevorzugt liegen dabei die beiden zweiten Lamellen 50 jeweils an den äußeren der drei ersten Lamellen 30 an, während die beiden dritten Lamellen 70 an der inneren der drei ersten Lamellen 30 anliegen und an ihrer äußeren Seitenkante jeweils an eine der beiden zweiten Lamellen 50 anliegen.

Die Lamellen 30, 50, 70 können entlang ihrer Lamellenlängsachse AL1, AL2, AL3 gekrümmt ausgebildet sein (vgl. insbesondere Figur 4). In diesem Fall kann die Durchgangsöffnung 24, 44 eine gekrümmte Anlagefläche 24a für die jeweilige Lamelle 50, 70 aufweisen (vgl. insbesondere Figur 5).

Die hohlrohrförmigen Grundkörper 20, 40 können wenigstens eine Spülöffnung 22, 42, vorzugsweise mehrere Spülöffnungen 22, 42, aufweisen.

An dem Retraktor 10, insbesondere an dem ersten Grundkörper 20, kann ein Aufnahmeelement 80 zur Aufnahme eines Halte- und/oder Manipulationsgriffs 90 angeordnet sein (vgl insbesondere Figur 7). Ist der Halte- und/oder Manipulationsgriff 90 in das Aufnahmeelement 80 eingesetzt, kann damit der Retraktor 10 durch einen Zugang in den Operationsbereich eingesetzt und an gewünschter Position und Ausrichtung positioniert werden.

### Bezugszeichenliste

- 10: Retraktor
- 20: erster Grundkörper
- 20a: Halbschale
- 20b: Halbschale
- 21: Stirnseite
- 22: Spülöffnung
- 24: Durchgangsöffnung
- 25: erste Transportstange
- 26: Vorsprung
- 27: Betätigungsabschnitt
- 30: erste Lamelle
- 30a: Seitenkante
- 31: erstes Ende
- 31: zweites Ende
- 33: Ausnehmung
- 35: Quersteg
- 40: zweiter Grundkörper
- 41: Stirnseite
- 42: Spülöffnung
- 44: Durchgangsöffnung
- 45: zweite Transportstange
- 46: Vorsprung
- 47: Betätigungsabschnitt
- 50: zweite Lamelle
- 50a: Seitenkante
- 51: erstes Ende
- 52: zweites Ende
- 53: Ausnehmung
- 54: Anschlagelement
- 60: dritter Grundkörper
- 70: dritte Lamelle
- 71: erstes Ende
- 72: zweites Ende
- 73: Ausnehmung
- 74: Anschlagelement
- 80: Aufnahmeelement
- 90: Halte- und/oder Manipulationsgriff
- lG1: Länge
- lG2: Länge
- lG3: Länge
- AG1: erste Grundkörperlängsachse
- AG2: zweite Grundkörperlängsachse
- AG3: dritte Grundkörperlängsachse
- AL1: erste Lamellenlängsachse
- AL2: zweite Lamellenlängsachse
- AL3: dritte Lamellenlängsachse
- AT1: erste Transportstangenlängsachse
- AT2: zweite Transportstangenlängsachse

## Patentansprüche

1. Chirurgischer Retraktor (10) für die Herzchirurgie mit einem hohlrohrförmigen ersten Grundkörper (20) mit einer ersten Grundkörperlängsachse (AG1), an welchem wenigstens eine erste Lamelle (30) aufweisend ein erstes Ende (31), ein zweites Ende (32) und eine erste Lamellenlängsachse (AL1) mit ihrem ersten Ende (31) seitlich angeordnet ist, sodass die erste Lamellenlängsachse (AL1) im Wesentlichen quer zu der ersten Grundkörperlängsachse (AG1) angeordnet ist, und mit einem zweiten Grundkörper (40) mit einer zweiten Grundkörperlängsachse (AG2), an welchem wenigstens eine zweite Lamelle (50) aufweisend ein erstes Ende (51), ein zweites Ende (52) und eine zweite Lamellenlängsachse (AL2) mit ihrem ersten Ende (51) seitlich angeordnet ist, sodass die zweite Lamellenlängsachse (AL2) im Wesentlichen quer zu der zweiten Grundkörperlängsachse (AG2) angeordnet ist, wobei in dem hohlrohrförmigen ersten Grundkörper (20) eine erste Transportstange (25) mit einer ersten Transportstangenlängsachse (AT1) um die erste Transportstangenlängsachse (AT1) drehbar gelagert angeordnet ist, wobei die wenigstens eine zweite Lamelle (50) den ersten Grundkörper (20) in einer Durchgangsöffnung (24) quer zu der ersten Grundkörperlängsachse (AG1) durchgreift und wobei die wenigstens eine zweite Lamelle (50) mehrere entlang der zweiten Lamellenlängsachse (AL2) beabstandet zueinander angeordnete Ausnehmungen (53) und die erste Transportstange (25) wenigstens einen mit den Ausnehmungen (53) zusammenwirkenden Vorsprung (26) aufweist, sodass bei Drehen der Transportstange (25) um ihre Transportstangenlängsachse (AT1) aufgrund des Zusammenwirkens des an der Transportstange (25) angeordneten Vorsprungs (26) mit den Ausnehmungen (53) der wenigstens einen zweiten Lamelle (50) die zweite Lamelle (50) durch die Durchgangsöffnung (24) bewegt wird, und, wenn in einer ersten Position der zweite Grundkörper (40) an dem ersten Grundkörper (20) anliegt, durch Drehen der Transportstange (25) die wenigstens eine zweite Lamelle (50) seitlich ausgefahren werden kann, wobei sich der Abstand zwischen dem zweiten Grundkörper (40) und dem ersten Grundkörper (20) vergrößert.

2. Chirurgischer Retraktor nach Anspruch 1,
**dadurch gekennzeichnet, dass** die erste Transportstange (25) mehrere Vorsprünge (26) aufweist, welche insbesondere nach Art eines Zahnrads ausgebildet sind.

3. Chirurgischer Retraktor nach einem der vorhergehenden Ansprüche,
**dadurch gekennzeichnet, dass** die erste Transportstange (25) einen unrunden Betätigungsabschnitt (27), vorzugsweise einen Betätigungsabschnitt (27) mit einem sechseckigen Querschnitt, aufweist, wobei vorzugsweise der Betätigungsabschnitt (27) an einer Stirnseite (21) des ersten Grundkörpers (20) aus dem Grundkörper (20) hervorragt.

4. Chirurgischer Retraktor nach einem der vorhergehenden Ansprüche,
**dadurch gekennzeichnet, dass** der erste Grundkörper (20) drei entlang der ersten Grundkörperlängsachse (AG1) beabstandet angeordnete erste Lamellen (30) aufweist.

5. Chirurgischer Retraktor nach einem der vorhergehenden Ansprüche,
**dadurch gekennzeichnet, dass** der zweite Grundkörper (40) zwei entlang der zweiten Grundkörperlängsachse (AG2) beabstandet angeordnete zweite Lamellen (50) aufweist.

6. Chirurgischer Retraktor nach einem der vorhergehenden Ansprüche,
**dadurch gekennzeichnet, dass** während einer Transportbewegung eine Seitenkante (50a) der wenigstens einen zweiten Lamelle (50) entlang einer Seitenkante (30a) der wenigstens einen ersten Lamelle (30) gleitend geführt ist, vorzugsweise nach Art einer Spundung.

7. Chirurgischer Retraktor nach einem der vorhergehenden Ansprüche,
**dadurch gekennzeichnet, dass** der zweite Grundkörper (40) eine Länge (lG2) aufweist, welche kleiner als eine Länge (lG1) des ersten Grundkörpers (20) ist, vorzugsweise um wenigstens 10% der Länge (lG1) des ersten Grundkörpers (20).

8. Chirurgischer Retraktor nach einem der vorhergehenden Ansprüche,
**dadurch gekennzeichnet, dass** ein dritter Grundkörper (60) mit einer dritten Grundkörperlängsachse (AG3) vorgesehen ist, an welchem wenigstens eine dritte Lamelle (70) aufweisend ein erstes Ende (71), ein zweites Ende (72) und eine dritte Lamellenlängsachse (AL3) mit ihrem ersten Ende (71) seitlich angeordnet ist, sodass die dritte Lamellenlängsachse (AL3) im Wesentlichen quer zu der dritten Grundkörperlängsachse (AG3) angeordnet ist, wobei der zweite Grundkörper (40) hohlrohrförmig ausgebildet ist und in dem hohlrohrförmigen zweiten Grundkörper(40) eine zweite Transportstange (45) mit einer zweiten Transportstangenlängsachse (AT2) um die zweite Transportstangenlängsachse (AT2) drehbar gelagert angeordnet ist, wobei die wenigstens eine dritte Lamelle (70) den zweiten Grundkörper (40) in einer Durchgangsöffnung (44) quer zu der zweiten Grundkörperlängsachse (AG2) durchgreift und wobei die wenigstens eine dritte Lamelle (70) mehrere entlang der dritten Lamellenlängsachse (AL3) beabstandet zueinander angeordnete Ausnehmungen (73) und die zweite Transportstange (45) wenigstens einen mit den Ausnehmungen (73) zusammenwirkenden Vorsprung (46) aufweist.

9. Chirurgischer Retraktor nach Anspruch 8,
**dadurch gekennzeichnet, dass** der dritte Grundkörper (60) zwei entlang der dritten Grundkörperlängsachse (AG3) beabstandet angeordnete dritte Lamellen (70) aufweist.

10. Chirurgischer Retraktor nach einem der vorhergehenden Ansprüche,
**dadurch gekennzeichnet, dass** die Breite einer Lamelle (30, 50, 70) etwa der Länge (lG1) des ersten Grundkörpers (20) geteilt durch die Anzahl sämtlicher Lamellen (30, 50, 70) des Retraktors (10) entspricht.

11. Chirurgischer Retraktor nach einem der vorhergehenden Ansprüche,
**dadurch gekennzeichnet, dass** die Lamellen (30, 50, 70) entlang ihrer Lamellenlängsachse (AL1, AL2, AL3) gekrümmt ausgebildet sind.

12. Chirurgischer Retraktor nach einem der vorhergehenden Ansprüche,
**dadurch gekennzeichnet, dass** die Durchgangsöffnung (24, 44) eine gekrümmte Anlagefläche (24a) für die Lamelle (50, 70) aufweist.

13. Chirurgischer Retraktor nach einem der vorhergehenden Ansprüche,
**dadurch gekennzeichnet, dass** die Lamellen (50, 70) an ihrem zweiten Ende (52, 72) ein vorzugsweise elastisch ausgebildetes Anschlagelement (54, 74) aufweisen.

14. Chirurgischer Retraktor nach einem der vorhergehenden Ansprüche,
**dadurch gekennzeichnet, dass** die hohlrohrförmigen Grundkörper (20, 40) wenigstens eine Spülöffnung (22, 42), vorzugsweise mehrere Spülöffnungen (22, 42), aufweisen.

15. Chirurgischer Retraktor nach einem der vorhergehenden Ansprüche,
**dadurch gekennzeichnet, dass** an dem ersten Grundkörper (20) ein Aufnahmeelement (80) zur Aufnahme eines Halte- und/oder Manipulationsgriffs (90) angeordnet ist.

## Claims

1. A surgical retractor (10) for cardiac surgery comprising a hollow-tubular first base body (20) with a first base body longitudinal axis (AG1) on which at least one first lamella (30) having a first end (31), a second end (32), and a first lamella longitudinal axis (AL1) is arranged laterally with its first end (31), such that the first lamella longitudinal axis (AL1) is arranged substantially transversely to the first base body longitudinal axis (AG1), and comprising a second base body (40) with a second base body longitudinal axis (AG2) on which at least one second lamella (50) having a first end (51), a second end (52), and a second lamella longitudinal axis (AL2) is arranged laterally with its first end (51), such that the second lamella longitudinal axis (AL2) is arranged substantially transversely to the second base body longitudinal axis (AG2), wherein a first transport rod (25) with a first transport rod longitudinal axis (AT1) is rotatably mounted about the first transport rod longitudinal axis (AT1) in the hollow-tubular first base body (20), wherein the at least one second lamella (50) extends through the first base body (20) in a through-opening (24) transverse to the first base body longitudinal axis (AG1), and wherein the at least one second lamella (50) has multiple recesses (53) spaced apart from each other along the second lamella longitudinal axis (AL2), and the first transport rod (25) has at least one projection (26) interacting with the recesses (53), such that, when the transport rod (25) is rotated about its transport rod longitudinal axis (AT1), the second lamella (50), due to the interaction of the projection (26) arranged on the transport rod (25) with the recesses (53) of the at least one second lamella (50), is moved through the through-opening (24), and, when in a first position the second base body (40) is in contact with the first base body (20), the at least one second lamella (50) can be extended laterally by rotating the transport rod (25), wherein the distance between the second base body (40) and the first base body (20) increases.

2. The surgical retractor according to claim 1,
**characterized in that** the first transport rod (25) has multiple projections (26) which are designed in particular in the manner of a gear wheel.

3. The surgical retractor according to any one of the preceding claims,
**characterized in that** the first transport rod (25) has a non-circular actuating section (27), preferably an actuating section (27) with a hexagonal cross-section, wherein preferably the actuating section (27) protrudes from the base body (20) at an end face (21) of the first base body (20).

4. The surgical retractor according to any one of the preceding claims,
**characterized in that** the first base body (20) has three first lamellae (30) spaced apart along the first base body longitudinal axis (AG1).

5. The surgical retractor according to any one of the preceding claims,
**characterized in that** the second base body (40) has two second lamellae (50) spaced apart along the second base body longitudinal axis (AG2).

6. The surgical retractor according to any one of the preceding claims,
**characterized in that**, during a transport movement, a side edge (50a) of the at least one second lamella (50) is guided sliding along a side edge (30a) of the at least one first lamella (30), preferably in the manner of a tongue and groove joint.

7. The surgical retractor according to any one of the preceding claims,
**characterized in that** the second base body (40) has a length (lG2) which is smaller than a length (lG1) of the first base body (20), preferably by at least 10% of the length (lG1) of the first base body (20).

8. The surgical retractor according to any one of the preceding claims,
**characterized in that** a third base body (60) with a third base body longitudinal axis (AG3) is provided, on which at least one third lamella (70) having a first end (71), a second end (72), and a third lamella longitudinal axis (AL3) is arranged laterally with its first end (71), such that the third lamella longitudinal axis (AL3) is arranged substantially transversely to the third base body longitudinal axis (AG3), wherein the second base body (40) is designed as a hollow tube and a second transport rod (45) with a second transport rod longitudinal axis (AT2) is rotatably mounted about the second transport rod longitudinal axis (AT2) in the hollow-tubular second base body (40), wherein the at least one third lamella (70) extends through the second base body (40) in a through-opening (44) transversely to the second base body longitudinal axis (AG2) and wherein the at least one third lamella (70) has multiple recesses (73) arranged apart from each other along the third longitudinal axis (AL3) of the lamella and the second transport rod (45) has at least one projection (46) that interacts with the recesses (73).

9. The surgical retractor according to claim 8,
**characterized in that** the third base body (60) has two third lamellae (70) spaced apart along the third base body longitudinal axis (AG3).

10. The surgical retractor according to any one of the preceding claims,
**characterized in that** the width of a lamella (30, 50, 70) corresponds approximately to the length (lG1) of the first base body (20) divided by the number of all lamellae (30, 50, 70) of the retractor (10).

11. The surgical retractor according to any one of the preceding claims,
**characterized in that** the lamellae (30, 50, 70) are curved along their longitudinal axis (AL1, AL2, AL3).

12. The surgical retractor according to any one of the preceding claims,
**characterized in that** the through-opening (24, 44) has a curved contact surface (24a) for the lamella (50, 70).

13. The surgical retractor according to any one of the preceding claims,
**characterized in that** the lamellae (50, 70) have a preferably elastically designed stop element (54, 74) on their second end (52, 72).

14. The surgical retractor according to any one of the preceding claims,
**characterized in that** the hollow-tubular base bodies (20, 40) have at least one rinsing opening (22, 42), preferably several rinsing openings (22, 42).

15. The surgical retractor according to any one of the preceding claims,
**characterized in that** a receiving element (80) for receiving a holding and/or manipulation handle (90) is arranged on the first base body (20).

## Revendications

1. Rétracteur (10) chirurgical pour la chirurgie cardiaque comprenant un premier corps (20) de base en forme de tube creux ayant un premier axe (AG1) longitudinal de corps de base, sur lequel est montée latéralement par sa première extrémité (31) au moins une première lamelle (30) ayant une première extrémité (31), une deuxième extrémité (32) et un premier axe (AL1) longitudinal de lamelle de manière à ce que le premier axe (AL1) longitudinal de lamelle soit sensiblement transversal au premier axe (AG1) longitudinal du corps de base et comprenant un deuxième corps (40) de base ayant un deuxième axe (AG2) longitudinal de corps de base sur lequel est montée latéralement par sa première extrémité (51) au moins une deuxième lamelle (50) ayant une première extrémité (51), une deuxième extrémité (52) et un deuxième axe (AL2) longitudinal de lamelle de manière à ce que le deuxième axe (AL2) longitudinal de la lamelle soit sensiblement transversal au deuxième axe (AG2) longitudinal du corps de base, dans lequel dans le premier corps (20) de base en forme de tube creux, une première barre (25) de transport ayant un premier axe (AT1) longitudinal de barre de transport est montée tournante autour du premier axe (AT1) longitudinal de barre de transport dans lequel la au moins une deuxième lamelle (50) traverse le premier corps (20) de base dans une ouverture (24) de passage, transversalement au premier axe (AG1) longitudinal du corps de base et dans lequel la au moins une deuxième lamelle (50) a plusieurs évidements (53) disposés à distance les uns des autres le long du deuxième axe (AL2) longitudinal de la lamelle et la première barre (25) de transport a au moins une saillie (26) coopérant avec les évidements (53) de manière à ce que, lorsque la barre (25) de transport tourne autour de son axe (AT1) longitudinal de barre de transport, en raison de la coopération de la saillie (26) disposée sur la barre (25) de transport avec les évidements (53) d'au moins une deuxième lamelle (50), la deuxième lamelle (50) se déplace dans l'ouverture (24) de passage et si dans une première position le deuxième corps (40) de base s'applique au premier corps (20) de base, par rotation de la barre (25) de transport la au moins une deuxième lamelle (50) peut s'écarter latéralement, la distance entre le deuxième corps (40) de base et le premier corps (20) de base étant agrandie.

2. Rétracteur chirurgical suivant la revendication 1,
**caractérisé en ce que** la première barre (25) de transport a plusieurs saillies (26), qui sont constituées en particulier à la manière d'une roue dentée.

3. Rétracteur chirurgical suivant l'une des revendications précédentes,
**caractérisé en ce que** la première barre (25) de transport a un tronçon (27) d'actionnement non circulaire, de préférence, un tronçon (27) d'actionnement de section transversale hexagonale, dans lequel de préférence, le tronçon (27) d'actionnement fait, d'un premier côté (21) frontal du corps (20) de base, saillie du corps (20) de base.

4. Rétracteur chirurgical suivant l'une des revendications précédentes,
**caractérisé en ce que** le premier corps (20) de base a trois première lamelles (30) disposées à distance le long de l'axe (AG1) longitudinal du premier corps de base.

5. Rétracteur chirurgical suivant l'une des revendications précédentes,
**caractérisé en ce que** le deuxième corps (40) de base a deux des deuxièmes lamelles (50) disposées à distance le long de l'axe (AG2) longitudinal du deuxième corps de base.

6. Rétracteur chirurgical suivant l'une des revendications précédentes,
**caractérisé en ce que**, pendant un mouvement de transport, un bord (50a) latéral de au moins une deuxième lamelle (50) est guidé à glissement le long d'un bord (30a) latéral de au moins une première lamelle (30), de préférence, à la manière d'un assemblage par rainure et languette.

7. Rétracteur chirurgical suivant l'une des revendications précédentes,
**caractérisé en ce que** le deuxième corps (40) de base a une longueur (lG2), qui est plus petite qu'une longueur (lG1) du premier corps (20) de base, de préférence, d'au moins 10% de la longueur (lG1) du premier corps (20) de base.

8. Rétracteur chirurgical suivant l'une des revendications précédentes,
**caractérisé en ce qu'**il est prévu un troisième corps (60) de base ayant un troisième axe (AG3) longitudinal de corps de base, sur lequel est disposée latéralement, par sa première extrémité (71), au moins une troisième lamelle (70) ayant une première extrémité (71), une deuxième extrémité (72) et un troisième axe (AL3) longitudinal de lamelle de manière à ce que le troisième axe (AL3) longitudinal de la lamelle soit sensiblement transversal au premier axe (AG3) longitudinal du troisième corps de base dans lequel le deuxième corps (40) de base est constitué sous la forme d'un tube creux et dans le deuxième corps (40) de base est montée, tournante autour d'un deuxième axe (AT2) longitudinal de barre de transport, une deuxième barre (45) de transport ayant le deuxième axe (AT2) longitudinal de transport dans lequel la au moins une troisième lamelle (70) traverse le deuxième corps (40) de base dans une ouverture (44) de passage, transversalement à l'axe (AG2) longitudinal du deuxième corps de base et dans lequel la au moins une troisième lamelle (70) a plusieurs évidements (73) disposés à distance les uns des autres le long de l'axe (AL3) longitudinal de la troisième lamelle et la deuxième barre (45) de transport a au moins une saillie (46) coopérant avec les évidements (73).

9. Rétracteur chirurgical suivant la revendication 8,
**caractérisé en ce que** le troisième corps (60) de base a deux troisièmes lamelles (70) disposées à distance le long de l'axe (AG3) longitudinal du troisième corps de base.

10. Rétracteur chirurgical suivant l'une des revendications précédentes,
**caractérisé en ce que** la largeur d'une lamelle (30, 50, 70) correspond à peu près à la longueur (lG1) du premier corps (20) de base divisée par le nombre de l'ensemble des lamelles (30, 50, 70) du rétracteur (10).

11. Rétracteur chirurgical suivant l'une des revendications précédentes,
**caractérisé en ce que** les lamelles (30, 50, 70) sont de constitution incurvées le long de leur axe (AL1, AL2, AL3) longitudinal de lamelle.

12. Rétracteur chirurgical suivant l'une des revendications précédentes,
**caractérisé en ce que** l'ouverture (24, 44) de passage a une surface (24a) incurvée d'application de la lamelle (50, 70).

13. Rétracteur chirurgical suivant l'une des revendications précédentes,
**caractérisé en ce que**
les lamelles (50, 70) ont, à leur deuxième extrémité (52, 72), un élément (54, 74) de butée, de préférence de constitution élastique.

14. Rétracteur chirurgical suivant l'une des revendications précédentes,
**caractérisé en ce que** les corps (20, 40) de base en forme de tube creux ont au moins une ouverture (22, 42) de lavage, de préférence, plusieurs ouvertures (22, 42) de lavage.

15. Rétracteur chirurgical suivant l'une des revendications précédentes,
**caractérisé en ce que**, sur le premier corps (20) de base, est disposé un élément (80) de réception d'une prise (90) de maintien et/ou de manipulation.
